# EUROPÄISCHE PATENTSCHRIFT

(11) **EP 1 318 191 B1**
(45) Veröffentlichungstag und Bekanntmachung des Hinweises auf die Patenterteilung: **14.06.2006**
(21) Anmeldenummer: 02026295.2
(22) Anmeldetag: 27.11.2002
(51) Int. Cl.: C11D 3/50, C11D 3/37, C11D 1/72, C11D 17/00, A61L 2/18, A61L 9/05

(54) **Haftende Paste zur Duftstoffabgabe, insbesondere für den Sanitärbereich**
Viscous paste for releasing fragrance, especially for use in sanitary installations
Pâte adhérente pour distribuer du parfum, notamment dans le domaine sanitaire

(30) Priorität: 06.12.2001 DE 10159984
(43) Veröffentlichungstag der Anmeldung: 11.06.2003
(73) Patentinhaber: Buck-Chemie GmbH ., 71083 Herrenberg (DE)
(72) Erfinder: Jaeschke, Edgr, Dipl.-Ing., 70794 Filderstadt (DE); Dettinger, Johannes, Dr., 72160 Horb am Neckar (DE); Seidel, Detlef, Dipl.-Ing., 71131 Jettingen (DE)
(74) Vertreter: Mammel, Ulrike

(56) Entgegenhaltungen:
- WO-A-01/19944
- WO-A-99/66017
- DE-A- 10 048 887
- DATABASE WPI Section Ch, Week 198822 Derwent Publications Ltd., London, GB; Class A97, AN 1988-150164 XP002057104 & JP 63 089598 A (HAKUGEN CO LTD), 20. April 1988 (1988-04-20)
- DATABASE WPI Section Ch, Week 199050 Derwent Publications Ltd., London, GB; Class A97, AN 1990-373633 XP002236779 & JP 02 272100 A (DAIICHI KOGYO SEIYAKU CO LTD), 6. November 1990 (1990-11-06)
- DATABASE WPI Section Ch, Week 199604 Derwent Publications Ltd., London, GB; Class A97, AN 1996-036110 XP002236780 & JP 07 305092 A (ST KAGAKU KK), 21. November 1995 (1995-11-21)

## Beschreibung

Die Erfindung betrifft eine haftende Paste zur Duftstoffabgabe, die insbesondere im Sanitärbereich einsetzbar ist.

Die Paste ist ein viskoses, im allgemeinen pastöses Gel, das aus einem entsprechenden Dosierbehältnis direkt auf der Oberfläche des Gegenstands wie eines Toilettenspülbeckens oder eines Handwaschbeckens aufgebracht wird, dort haftet und sich erst nach einer größeren Anzahl von Spülvorgängen nach und nach auflöst.

Durch Zugabe von Tensiden ist die Duftstoffpaste auch als Sanitärreinigungsmittel einsetzbar.

Durch die direkte Haftung der Paste auf der Oberfläche des Sanitärgegenstandes ist es nicht erforderlich, zusätzlich Behältnisse wie die sogenannten "WC-Körbchen" vorzusehen, deren Benutzung vom Verbraucher insbesondere beim Ersetzen des Sanitärmittels und bei der Reinigung der Toilette als unhygienisch empfunden wird.

Aus der WO 99/66017 und der DE 100 48 887 sind bereits haftende Sanitärmittel zur Duftstoffabgabe, Reinigung und Desinfektion bekannt, die direkt auf die Sanitärgegenstände applizierbar sind. Diese Mittel umfassen Duftstoffe, Wasser, Haftvermittler und Tenside und sind viskose, feste oder pastöse Mittel.

Die bekannten haftenden Sanitärmittel lassen sich auf einfache und hygienische Art und Weise mit einer geeigneten Vorrichtung applizieren, haften an der Oberfläche des Sanitärgegenstands, behalten ihre Form bei und werden selbst unter Einwirkung von Wasser nicht als Ganzes abgespült, sondern erst nach einer Vielzahl von Spülungen vollständig aufgelöst.

Der vorliegenden Erfindung liegt die Aufgabe zugrunde, Pasten zur Duftstoffabgabe bereitzustellen, die insbesondere im Sanitärbereich eingesetzt werden können.

Diese Aufgabe wird durch die Merkmale des Anspruchs 1 gelöst.

Erstaunlicherweise wurde festgestellt, dass eine haftende und sich erst nach einer größeren Anzahl von Spülvorgängen nach und nach auflösende Paste zur Duftstoffabgabe bereitgestellt werden kann, wenn die Paste als Haftvermittler Blockcopolymere, die verknüpfte Oligomere bestehend aus Oligo- oder Polyethylenoxid und/oder Oligo- oder Polypropylenoxid und/oder Oligo- oder Polybutylenoxid umfassen, oder Aryl-Ethoxylate oder Alkyl-Aryl-Ethoxylate sowie Wasser und Parfüm umfasst.

Der Haftvermittler bewirkt in Anwesenheit von Wasser, dass das Mittel an der Oberfläche haftet. Im allgemeinen bildet er netzwerkartige Strukturen aus, die dem Mittel selbst unter der starken Krafteinwirkung durch Spülwasser die erforderliche Formbeständigkeit verleihen.

Die Moleküle des Haftvermittlers sind länger- oder langkettige, im wesentlichen gestreckte Moleküle, die wenigstens teilweise hydrophil sind und somit wenigstens einen hydrophilen Rest oder eine hydrophile Gruppe umfassen, die mit Wasser wechselwirkt.

Im Rahmen der vorliegenden Erfindung werden unter Blockcopolymer Makromoleküle aus linear miteinander chemisch verknüpften Blöcken aus mehreren Monomeren verstanden. Vorzugsweise umfassen diese Blockcopolymere wenigstens einen Oligo- oder Polyethylenoxidblock. Weiterhin umfasst das Blockcopolymer ein weiteres Polymer, wobei Oligo- oder Polypropylenoxid und/oder Oligo- oder Polybutylenoxid als Copolymere im Rahmen der vorliegenden Erfindung bevorzugt sind. Eine erstaunlich gute Haftung und Formstabilität wird mit EO-PO-EO-Blockpolymeren der folgenden Formel HO-(CH₂-CH₂-O)ₓ-(CHCH₃-CH₂₋O)_{y}(-CH₂-CH₂-O)_{z}-H erzielt. Vorzugsweise beträgt x > 2, y > 20, z > 2. Bei geringen Molmassen des PO-Blocks im Molekül ist eine hohe Molmasse der EO-Blöcke notwendig. Mit steigender Molmasse des Polypropylenoxid-Blockes kann bei guter Haftung, Formstabilität und Beibehaltung der gewünschten Abspülbarkeit die Molmasse der EO-Blöcke verringert werden. Beträgt die Molmasse des PO-Blockes zum Beispiel im Blockpolymer 1750 g/mol, so sollte der Anteil des EO-Blockes beziehungsweise der EO-Blöcke im Molekül vorzugsweise > 40 Gew.% betragen. Bei einer Molmasse des PO-Blockes von zum Beispiel 3250 g/mol kann der Anteil des EO-Blockes beziehungsweise der EO-Blöcke bis auf 10 Gew.% reduziert werden. Je höher die Molmasse der EO-Blöcke im Molekül ist, um so ausgeprägter ist die Gelbildung. Eine besonders gute Gelbildung wird erreicht, wenn jeweils die Enden des Blockcopolymers EO-Blöcke aufweisen.

Anstelle des PO-Blockes kann auch ein B(utylen)O-Block verwendet werden.

Ebenfalls wurde festgestellt, dass haftende, abspülbare und pastöse Gele zur Duftstoffabgabe bereitgestellt werden können, wenn diese Aryl-Ethoxylate bzw. Alkyl-Aryl-Ethoxylate als Haftvermittler umfassen. Die Ausprägung zur Gelbildung nimmt dabei mit höherer Anzahl an Ethoxygruppen in dem Molekül zu. Vorzugsweise weisen die Alkyl-Aryl-Ethoxylate bzw. die Aryl-Ethoxylate zwischen 3 und 50, vorzugsweise zwischen 6 und 30, EO-Gruppen auf. Wird ein Alkyl-Aryl-Ethoxylat eingesetzt, so umfasst der Alkylrest vorzugsweise zwischen 1 und 50, besonders bevorzugt 8 bis 22, Kohlenstoffatome, wobei lineare Alkylreste bevorzugt sind. Besonders bevorzugt ist, p-Alkylphenylethoxylat einzusetzen, das einen C₅-C₅₀-Rest, insbesondere einen linearen C₅-C₁₅-Rest, aufweist. Besonders bevorzug ist, Nonylphenolethoxylat mit 20 EO einzusetzen.

Als Aryl-Ethoxylate kann beispielsweise das Imbentin BN/150 der Firma Dr. W. Kolb AG ausgewählt werden.

Vorzugsweise sollte es sich bei den Haftvermittlern um unverzweigte Moleküle handeln, um die gewünschte Netzwerkbildung zu ermöglichen.

Unter "Parfüm" werden im Rahmen der vorliegenden Erfindung Parfüm, Parfümöl und sonstige Riech- und Beduftungsmittel verstanden. Vorzugsweise sind die Parfüms flüssige ölige Substanzen. Der prozentuale Anteil an Parfüm beträgt in der erfindungsgemäßen Paste zwischen 1 und 20 Gew.%, wobei ein Parfümgehalt von etwa 5 bis 15 Gew.% bevorzugt ist.

Die erfindungsgemäße Paste kann durch Zugabe weiterer Bestandteile auch zur Reinigung, Entkaltung oder Desinfektion eingesetzt werden.

Als Tenside können prinzipiell alle bekannten anionischen und/oder nichtionischen und/oder kationischen und/oder amphoteren Tenside eingesetzt werden.

Gute Ergebnisse wurden mit Polyalkoxyalkanen, vorzugsweise einem Gemisch aus Alkylethoxylaten mit 20 - 40 EO, vorzugsweise 30 und 35 EO, und Alkyl = C16, C18, C20, C22 als nichtionischem Tensid erzielt.

Als anionisches Tensid, das gleichzeitig als Schäumer fungiert, hat sich insbesondere Alkyl(C12-C14)-polyethylenglykol-(2EO)-ethersulfat-trüsopropanolammoniumsalz bewährt. Dieses anionische Tensid ist bevorzugt, da - im Gegensatz zu Olefinsulfonat oder Taurid - die Paste nicht zur Synhärese neigt.

Durch die Zugabe von Di-, Oligo- oder Polyhydroxyverbindungen oder deren Ether wird die Feuchtigkeit der Oberfläche des haftenden Sanitärmittels erhalten, so dass das aufgebrachte Mittel auch bei einer geringen Spülfrequenz eine glatte, im wesentlichen transparente Oberfläche beibehält.

Die Di-, Oligo- oder Polyhydroxyverbindungen oder deren Ether dienen gleichzeitig als Lösungsvermittler und bewirken eine Beschleunigung des Auflösevorganges des Mittels und damit eine konstante Freisetzung der einzelnen Bestandteile des Sanitärmittels in konzentrierter Form bei jedem Spülvorgang. Das Abspülverhalten des Mittels wird bei Verwendung der Feuchthaltemittel gegenüber der Verwendung von Natriumsulfat, das beispielsweise in der WO 99/66017 eingesetzt wird, deutlich verbessert. Auch auf die Konsistenz des Mittels haben die erfindungsgemäßen Feuchthaltemittel einen positiven Einfluss.

Als aliphatische Di-, Oligo- oder Polyhydroxyverbindung oder deren Ether kann jede aliphatische Verbindung eingesetzt werden, die wenigstens zwei Hydroxygruppen aufweist, beispielsweise Glykol, 1,2- oder 1,3 Dihydroxypropan, 1,2-, 1,3-, 2,3- oder 1,4-Dihydroxybutan, die Isomeren des Dihydroxyisobutans, die Dihydroxypentane etc. Auch Trihydroxyverbindungen, wie beispielsweise Glycerin oder auch längerkettige Trihydroxyverbindungen, wie beispielsweise Trihydroxyhexan, Trihydroxyhepten etc., können als Feuchthaltemittel eingesetzt werden. Als Tetrahydoxyverbindung kann zum Beispiel Pentaerythrit eingesetzt werden. Als weitere Oligohydroxyverbindungen kommen Pentahydroxy-, Hexahydroxyverbindungen etc. in Betracht. Auch Zucker, Polysaccharide, wie Stärke, Cellulose oder Polymere sind als Oligo- oder Polyhydroxyverbindungen einsetzbar. Es ist ebenfalls möglich, als Feuchthaltemittel Ether der Di-, Oligo- oder Polyhydroxyverbindungen einzusetzen, wie beispielsweise Diethylenglykol, Oligoethylenglykole wie das Triethylenglykol, aber auch Polyhydroxyether wie beispielsweise Polymethylen- oder Polyethylenglykol.

Um die gewünschte Feuchtigkeitsregulierung zu erreichen, kann die Paste zwischen 1 und 20 Gew.%, vorzugsweise zwischen 5 und 15 Gew.% und besonders bevorzugt zwischen 7 und 13 Gew.%, der aliphatischen Di-, Oligo- oder Polyhydroxyverbindungen oder deren Ether aufweisen. Besonders bevorzugt ist, als Feuchthaltemittel Glycerin einzusetzen.

Auch kann die Paste weitere übliche Bestandteile umfassen, beispielsweise Desinfektionsmittel, Konservierungsstoffe, wie zum Beispiels Isothiazolon-Derivate oder Schaumstabilisatoren, wie Kokosfettsäureamidopropylbetain oder Kokosfettsäureamidopropyldimethylaminoxid, Kokosfettsäuremono/diethanolamid oder Alkylpolyetherglycerolethersulfate, aber auch Farbstoffe und/oder Kalk- oder Urinstein lösende Substanzen, insbesondere Säuren und/oder Verdicker.

Der Paste können darüber hinaus - falls gewünscht - auch Salze, wie beispielsweise Natriumsulfat, hinzugegeben werden, um die Auflösegeschwindigkeit zu erhöhen. Der Salzanteil kann bis zu 10 Gew.%, vorzugsweise bis zu 5 Gew.%, betragen.

Nachfolgend sind zwei Rahmenrezepturen für die erfindungsgemäße Paste mit weiteren Bestandteilen aufgeführt. Die Rahmenrezeptur 1 betrifft eine erfindungsgemäße Paste mit Alkyl-Aryl- oder Arylethoxylaten als Haftvermittler, die Rahmenrezeptur 2 eine Paste mit Blockcopolymeren mit wenigstens einem Oligo-Ethylenoxidblock als Haftvermittler:

| Bestandteil | Gehalt (%) Rahmenrezeptur 1 | Gehalt (%) Rahmenrezeptur 2 |
|---|---|---|
| Alkyl-Aryl oder Arylethoxylat, insbesondere Nonylphenolethoxylate | 20 | - |
| Blockcopolymere mit Oligo-Ethylenoxid-Block oder Blöcken, insbesondere EO-PO-EO-Blockcopolymere | - | 20-50 |
| Alkylethersulfat als anionische Tenside | 0 - 10 | 0 - 10 |
| nichtionische Tenside, insbesondere Alkylethoxylate | 0 - 10 | 0 - 10 |
| Polyethylenglykol | 0 - 3 | 0 - 3 |
| Di-, Oligo- oder Polyhydroxiverbindungen oder deren Ether | 0 - 10 | 0 - 10 |
| Farbe | 0 - 1 | 0 - 1 |
| Konservierungsmittel | 0 - 2 | 0 - 2 |
| Verdicker | 0 - 2 | 0 - 2 |
| Parfümöl | 1 - 20 | 1 - 20 |
| Wasser | ad 100 | ad 100 |

Die erfindungsgemäße Paste kann in hygienischer Weise ohne Berührung von mit dem WC-Becken verbundenen, möglicherweise verunreinigten Vorrichtungen aufgebracht und erneuert werden.

Ein wesentlicher Vorteil der erfindungsgemäßen Paste besteht darin, dass sie nach den Wünschen des Verbrauchers portionierbar ist. Wünscht der Verbraucher eine intensivere Beduftung oder wird beispielsweise die Toilette häufig benutzt, so kann entsprechend stärker dosiert werden.

Auch kann auf einem Sanitärgegenstand an verschiedenen Orten das erfindungsgemäße Mittel in unterschiedlichen Zusammensetzungen appliziert werden. Dies ermöglicht beispielsweise, dass zwei untereinander unverträgliche Inhaltsstoffe, beispielsweise halogenfreisetzende Mittel und oxidationsempfindliche Parfümierungsstoffe, durch örtliche Trennung dennoch zu einer gemeinsamen Reinigung und/oder Beduftung der Toilette eingesetzt werden können.

Die erreichte Haftung ist an dem Sanitärgegenstand selbst bei einer Anbringung an einer senkrechten Fläche so gut, dass sich das Mittel sogar unter der zusätzlichen Krafteinwirkung von Spülwasserströmen nicht ablöst.

Die erfindungsgemäße Paste löst sich erst mit einer größeren Anzahl von Spülvorgängen nach und nach auf. Die Anzahl der Spülvorgänge richtet sich natürlich nach der Zusammensetzung der jeweiligen Paste, der aufgebrachten Menge sowie der Geometrie des aufgebrachten Pastenstrangs.

Die Paste lässt sich auch für andere Sanitärgegenstände einsetzen wie Urinale, Pissoirs, aber auch für Handspülbecken oder dergleichen. Auch kann die Paste beispielsweise auf Duschtassen aufgebracht werden.

Vorzugsweise ist die erfindungsgemäße Paste ein salbenartiges pastöses und/oder cremeartiges Gel. Die Gele sind im wesentlichen formstabil und standfest über einen längeren Zeitraum, so dass sie nicht "herunterlaufen" oder "tropfen".

Die Gele lassen sich vorzugsweise über Tuben, vergleichbar den Zahnpastatuben, Spritzen oder Kartuschen in das Toilettenbecken einbringen und bleiben dort für eine Vielzahl von aufeinanderfolgenden Spülvorgängen haften.

Die an einem Haake-Viskosimeter, System Platte-Kegel, Sensor PK 1 1°, Schergefälle von 17,8 und 30 s⁻¹ und Raumtemperatur (20° C) zu bestimmenden Viskositäten der erfindungsgemäßen Paste sollten wenigstens 15.000 mPas, üblicherweise wenigstens 50.000 mPas, vorzugsweise wenigstens 90.000 mPas und besonders bevorzugt wenigstens 110.000 mPas, betragen.

Die Erfindung wird nachfolgend anhand von Ausführungsbeispielen beschrieben.

### 1. Rezeptur einer erfindungsgemäßen Duftpaste

| Bestandteil | Gehalt (Gew.%) | Lieferant |
|---|---|---|
| EO/PO/EO-Blockcopolymer mit 80 % Polyethylenoxidanteil im Molekül (Pluronic PE 6800) | 50 | (BASF AG) |
| Parfümöl Citrix Extra F548373 | 10 | (Quest International) |
| Wasser | ad 100 | |

Die erfindungsgemäße Paste ist standfest, zeigt keine Synhärese und schäumt beim Überspülen mit Wasser schwach. Die dem oben beschriebenen Haake-Viskosimeter bei 20°C gemessene Viskosität beträgt 50.000 mPas bei 17,8 s⁻¹ und 30.000 mPas bei 30 s⁻¹.

Beim Auftragen von 5 ml Masse mit einer Strangbreite von circa 1,3 cm wurde bei der erfindungsgemäßen Paste gemäß Ausführungsbeispiel 1 Spülzahlen von > 150 ermittelt.

### 2. Rezeptur einer weiteren Duftpaste

| Bestandteil | Gehalt (Gew.%) | Lieferant |
|---|---|---|
| Polyoxyethylen-Polyoxypropylen-Blockpolymer (Lutrol F 127) | 30 | (BASF AG) |
| Rewominox B 204 Aminoxid | 2,5 | (Franken-Chemie) |
| Parfümöl Fresh & Clean | 20 | (Quest International) |
| Wasser | ad 100 | |

Die erfindungsgemäße Paste ist standfest, zeigt keine Synhärese und schäumt schwach . Die dem oben beschriebenen Haake-Viskosimeter bei 20°C gemessene Viskosität beträgt bei 17,8 s⁻¹ 185.000 mPas und bei 30 s⁻¹ 56.000 mPas.

Beim Auftragen von 5 ml Masse mit einer Strangbreite von circa 1,3 cm wurde bei der erfindungsgemäßen Paste gemäß Ausführungsbeispiel 1 Spülzahlen von > 150 ermittelt.

### 3. Duftstoffpaste mit Nonylphenolethoxylat

| Bestandteil | Gehalt (Gew.%) | Lieferant |
|---|---|---|
| Nonylphenolethoxylat mit 20 EO (Lutensol AP 20) | 25 | (BASF AG) |
| Parfümöl Citrix Extra F 548373 | 10 | (Quest International) |
| Wasser | ad 100 | |

Die erfindungsgemäße Paste ist standfest, zeigt keine Synhärese und schäumt beim Überspülen mit Wasser. Die mit dem oben beschriebenen Haake-Viskosimeter bei 20°C gemessene Viskosität der pastösschnittfähigen Paste beträgt bei 17,8 s⁻¹ 110.000 mPas und bei 30 s⁻¹ 70.000 mPas.

Beim Auftragen von 5 ml Masse mit einer Strangbreite von circa 1,3 cm wurde bei der erfindungsgemäßen Paste gemäß Ausführungsbeispiel 1 Spülzahlen von > 100 ermittelt.

## Patentansprüche

1. Paste zur Duftstoffabgabe insbesondere für den Sanitärbereich, welche Paste unmittelbar auf den Gegenstand, insbesondere einen Sanitärgegenstand, applizierbar ist, dort haftet und sich erst nach einer größeren Anzahl von Spülvorgängen nach und nach auflöst, wobei die Paste
einen Haftvermittler aus der Gruppe der Oligo- oder Polyethylenoxid und/oder Oligo- und/oder Polypropylenoxid und/oder Oligo- und/oder Polybutylenoxid umfassenden Blockcopolymere, der Aryl-Ethoxylate oder Alkyl-Aryl-Ethoxylate umfasst sowie Parfüm und Wasser.

2. Paste nach Anspruch 1, **dadurch gekennzeichnet, dass** die Oligo- oder Polyethylenoxid umfassenden Blockpolymere zusätzlich Oligo- oder Polypropylenoxid und/oder Oligo- oder Polybutylenoxid umfassen.

3. Paste nach Anspruch 2, **dadurch gekennzeichnet, dass** das Blockpolymer ein EO-PO-EO-Blockcopolymer ist.

4. Paste nach Anspruch 3, **dadurch gekennzeichnet, dass** bei den EO-PO-EO-Blockcopolymeren der Formel HO-(CH₂-CH₂-O)ₓ-(CHCH₃-CH₂₋O)_{y}(-CH₂-CH₂-O)_{z}-H x > 2, y > 20 und z > 2 ist.

5. Paste nach Anspruch 1, **dadurch gekennzeichnet, dass** das Alkyl-Aryl-Ethoxylat p-Alkylphenylethoxylat mit Alkyl = C₁-C₅₀ ist und die Verbindung vorzugsweise zwischen 3 und 50, vorzugsweise zwischen 6 und 30, EO-Gruppen umfasst.

6. Paste nach einem der vorangegangenen Ansprüche, **dadurch gekennzeichnet, dass** sie zwischen 10 und 60 Gew.% des Blockpolymers oder des Aryl- oder Alkyl-Aryl-Ethoxylats und zwischen 1 und 25 Gew.% Parfüm umfasst.

7. Paste nach einem der vorangegangenen Ansprüchen, **dadurch gekennzeichnet, dass** die Paste weiterhin anionische, nichtionische, kationische oder amphothere Tenside und/oder Desinfektionsmittel und/oder Konservierungsstoffe und/oder Farbstoffe und/oder Kalk-oder Urinstein lösende Substanzen und/oder Salze umfasst.

8. Paste nach einem der vorangegangenen Ansprüche, **dadurch gekennzeichnet, dass** sie umfasst:
15 - 50 Gew.% Aryl-Ethoxylat, Alkyl-Aryl-Ethoxylate oder Blockcopolymere
0 - 10 Gew.% anionische Tenside
0 - 10 Gew.% nichtionische Tenside
0 - 3 Gew.% Polyethylenglykol
0 - 10 Gew.% Feuchthaltemittel, insbesondere Glycerin
0 - 1 Gew.% Farbe
0 - 2 Gew.% Konservierungsmittel
0 - 2 Gew.% Verdicker
2 - 20 Gew.% Parfüm
Wasser ad 100.

9. Paste nach einem der vorangegangenen Ansprüche, **dadurch gekennzeichnet, dass** die mit einem Haake-Viskosimeter, System Platte-Kegel, Sensor PK 1 1°, Schergefälle von 17,8 s⁻¹ beziehungsweise 30 s⁻¹ und 20°C bestimmte Viskosität wenigstens 15.000 mPas, vorzugsweise wenigstens 50.000 mPas und besonders bevorzugt wenigstens 110.000 mPas beträgt.

10. Paste nach Anspruch 1, **dadurch gekennzeichnet, dass** die Paste in Form eines Geles vorliegt.

11. Verfahren zur Applikation einer Paste nach Anspruch 1, **dadurch gekennzeichnet, dass** die Paste direkt auf die Oberfläche des Gegenstands, insbesondere des Sanitärgegenstands, aufgebracht wird und dort haftet.

## Claims

1. Paste for releasing fragrance, especially for the sanitary sector, which paste can be applied directly, to the object, in particular a sanitary object, adheres there and dissolves gradually only after a relatively large number of flushing operations, where the paste comprises an adhesion promoter from the group of block copolymers comprising oligo- or polyethylene oxide and/or oligo- and/or polypropylene oxide and/or oligo-and/or polybutylene oxide, of aryl ethoxylates or alkylaryl ethoxylates, and also perfume and water.

2. Paste according to Claim 1, **characterized in that** the block polymers comprising oligo- or polyethylene oxide additionally comprise oligo- or polypropylene oxide and/or oligo- or polybutylene oxide.

3. Paste according to Claim 2, **characterized in that** the block polymer is a EO-PO-EO block copolymer.

4. Paste according to Claim 3, **characterized in that** in the case of the EO-PO-EO block copolymers of the formula HO- (CH₂-CH₂-O)ₓ- (CHCH₃-CH₂-O)_{y}(-CH₂-CH₂-O)_{z}-H, x is > 2, y is > 20 and z is > 2.

5. Paste according to Claim 1, **characterized in that** the alkylaryl ethoxylate is p-alkylphenyl ethoxylate where alkyl = C₁-C₅₀ and the compound preferably comprises between 3 and 50, preferably between 6 and 30, EO groups.

6. Paste according to one of the preceding claims, **characterized in that** it comprises between 10 and 60% by weight of the block polymer or of the aryl ethoxylate or alkylaryl ethoxylate and between 1 and 25% by weight of perfume.

7. Paste according to one of the preceding claims, **characterized in that** the paste further comprises anionic, nonionic, cationic or amphoteric surfactants and/or disinfectants and/or preservatives and/or dyes and/or substances which dissolve lime or urine scale and/or salts.

8. Paste according to one of the preceding claims, **characterized in that** it comprises:
15-50% by weight of aryl ethoxylate, alkylaryl ethoxylates or block copolymers
0-10% by weight of anionic surfactants
0-10% by weight of nonionic surfactants
0-3% by weight of polyethylene glycol
0-10% by weight of humectants, in particular glycerol
0-1% by weight of dye
0-2% by weight of preservative
0-2% by weight of thickener
2-20% by weight of perfume
water ad 100.

9. Paste according to one of the preceding claims, **characterized in that** the viscosity determined using a Haake viscometer, plate-cone system, sensor PK 1 1°, shear drop of 17.8 s⁻¹ or 30 s⁻¹ and 20°C is at least 15 000 mPas, preferably at least 50 000 mPas and particularly preferably at least 110 000 mPas.

10. Paste according to Claim 1, **characterized in that** the paste is in the form of a gel.

11. Method of applying a paste according to Claim 1, **characterized in that** the paste is applied directly to the surface of the object, in particular the sanitary object, and adheres there.

## Revendications

1. Pâte pour la libération d'un parfum, en particulier pour le domaine sanitaire, ladite pâte pouvant être appliquée directement sur un objet, en particulier un objet sanitaire, y adhérant et n'étant détachée petit à petit qu'après un assez grand nombre de processus de rinçage, la pâte contenant un promoteur d'adhérence du groupe des copolymères à blocs, comprenant de l'oligo(oxyde d'éthylène) ou du poly(oxyde d'éthylène) et/ou de l'oligo(oxyde de propylène) et/ou du poly(oxyde de propylène) et/ou de l'oligo(oxyde de butylène) et/ou du poly-(oxyde de butylène), des éthoxylates d'aryle ou des éthoxylates d'alkylaryle ainsi que du parfum et de l'eau.

2. Pâte selon la revendication 1, **caractérisée en ce que** les polymères à blocs comprenant de l'oligo(oxyde d'éthylène) ou du poly(oxyde d'éthylène) comprennent en outre de l'oligo(oxyde de propylène) et/ou du poly(oxyde de propylène) et/ou de l'oligo(oxyde de butylène) ou du poly(oxyde de butylène).

3. Pâte selon la revendication 2, **caractérisée en ce que** le polymère à blocs est un copolymère à blocs d'OE-OP-OE.

4. Pâte selon la revendication 3, **caractérisée en ce que** dans le cas des copolymères à blocs d'OE-OP-OE de formule HO-(CH₂-CH₂-O)ₓ-(CHCH₃-CH₂-O)_{y}(-CH₂-CH₂-O)_{z}-H, x > 2, y > 20 et z > 2.

5. Pâte selon la revendication 1, **caractérisée en ce que** l'éthoxylate d'alkylaryle est un éthoxylate de p-alkylphényle avec alkyle = C₁-C₅₀ et le composé comprend de préférence entre 3 et 50, de préférence entre 6 et 30, groupes d'OE.

6. Pâte selon l'une quelconque des revendications précédentes, **caractérisée en ce qu'**elle comprend entre 10 et 60% en poids du polymère à blocs ou de l'éthoxylate d'aryle ou d'alkylaryle et entre 1 et 25% en poids de parfum.

7. Pâte selon l'une quelconque des revendications précédentes, **caractérisée en ce que** la pâte comprend en outre des agents tensioactifs anioniques, non ioniques, cationiques ou amphotères et/ou des désinfectants et/ou des conservateurs et/ou des colorants et/ou des substances dissolvant le calcaire ou la pierre d'urine et/ou des sels.

8. Pâte selon l'une quelconque des revendications précédentes, **caractérisée en ce qu'**elle comprend :
15 - 50% en poids d'éthoxylate d'aryle, d'éthoxylate d'alkylaryle ou de copolymères à blocs,
0 - 10% en poids d'agents tensioactifs anioniques,
0 - 10% en poids d'agents tensioactifs non ioniques,
0 - 3% en poids de polyéthylèneglycol,
0 - 10% en poids d'agents de rétention de l'humidité, en particulier le glycérol,
0 - 1% en poids de colorant,
0 - 2% en poids de conservateur,
0 - 2% en poids d'épaississant,
2 - 20% en poids de parfum,
eau ad 100.

9. Pâte selon l'une quelconque des revendications précédentes, **caractérisée en ce qu'**elle présente une viscosité, déterminée avec un viscosimètre de Haake, système plaque-cône, sonde PK 1 1°, gradient de cisaillement de 17,8 s⁻¹ ou de 30 s⁻¹ et 20°C, d'au moins 15 000 mPa.s, de préférence d'au moins 50 000 mPa.s et de manière particulièrement préférée d'au moins 110 000 mPa.s.

10. Pâte selon la revendication 1, **caractérisée en ce que** la pâte se trouve sous forme d'un gel.

11. Procédé pour l'application d'une pâte selon la revendication 1, **caractérisé en ce que** la pâte est appliquée directement sur la surface de l'objet, en particulier l'objet sanitaire, et y adhère.
